Europäisches Patentamt

(19) European Patent Office

Office européen des brevets

(11) Publication number : **0 340 662 B1**

# (12) EUROPEAN PATENT SPECIFICATION

(45) Date of publication of patent specification :
24.07.91 Bulletin 91/30

(51) Int. Cl.⁵ : **A61K 31/195, A61K 9/16**

(21) Application number : **89107755.4**

(22) Date of filing : **28.04.89**

(54) **Solid, water-soluble, pharmaceutical composition containing acetylcysteine.**

(30) Priority : 05.05.88 CH 1681/88

(43) Date of publication of application :
08.11.89 Bulletin 89/45

(45) Publication of the grant of the patent :
24.07.91 Bulletin 91/30

(84) Designated Contracting States :
AT BE CH DE ES FR GB GR IT LI LU NL SE

(56) References cited :
DE-A- 1 961 769
DE-A- 3 441 862
GB-A- 2 192 789
DICTIONNAIRE VIDAL 1986, pages
570,599,969, O.V.P., Paris, FR

(56) References cited :
RESEARCH DISCLOSURE, vol. 151, November
1976, page 5, disclosure no. 15102, Industrial
Opportunities Ltd, Havant, GB;
"Kalorienarmes, süsshaltiges Zuckeraus-
tauschmittel"

(73) Proprietor : IBSA - INSTITUT BIOCHIMIQUE
S.A.
Via al Ponte, 13
CH-6903 Lugano (CH)

(72) Inventor : Lualdi, Paolo
Via Baradello
F-22070 Grandate (Como) (IT)

(74) Representative : Gervasi, Gemma et al
NOTARBARTOLO & GERVASI Srl Viale Bianca
Maria 33
I-20122 Milan (IT)

## Description

The present invention relates to a solid, water-soluble, pharmaceutical composition having as active principle acetylcysteine.

It is known that acetylcysteine (see Index Nominum—1987—page 9) is a highly tolerable drug possessing interesting pharmacological activities, predominant among which the mucolytic one.

It is also known that acetylcysteine may be administered through all the customary administration methods.

It is further known that the administration of acetylcysteine as a mucolytic is frequently done "per os" using solid water-soluble pharmaceutical forms, which are dissolved in water at the moment of use, such as e.g. powders, granules or effervescent tablets.

Similarly to many aliphatic thioderivatives, acetylcysteine has disagreable odor and taste, which must be adequately masked. In the past many formulations have been suggested in which attempts were made to mask the taste and odor of acetylcysteine by the addition of sweetening and flavoring agents.

The problem of the palatability may be solved in a relatively satisfactory way by using saccharose as a sweetening agent ; however, the amount required is rather high and inconvenient to diabetic patients as well as in all other instances in which it is desirable to limit the assumption of high caloric substances.

Other sweetening agents do not have a sufficient masking capacity.

In particular GB-A-2 192 789 refers to a pharmaceutical composition in the form of water-soluble granules containing :

| | |
|---|---|
| N-acetylcysteine | (10-20% by weight) |
| Aspartame | (2-3% by weight) |
| Sorbitol | (67/78% by weight) |
| Flavouring Agent | (about 10% by weight) |

This document stresses the fact (see pag.1, lines 17-24) that the substitution of a sweetening agent with another is not an easy problem to solve.

DE-A-3 441 862 describes various compositions containing sugar substituents and artificial sweetening agents.

No indication is given that a combination similar to the one used in the present invention is suitable to solve the present problem (i.e. the disagrable odor and taste of acetylcysteine).

Dictionnaire Vidal reports three different compositions having mucolytic activity and containing acetylcysteine wherein the sweetening factors are saccharine or saccharine and saccharose. No reference is made to the possible use of xylitol as sweetening agent for this kind of composition. DE-A-1 961 769 deals with sweetening compositions containing a sugar substituent and saccharine in ratios comprised between 50 : 50 and 90 : 10. In this connection, and also referring to what already said above for the first document (i.e. GB-A-2 192 789), it is interesting to note that the document Research Disclosure vol.151 n. 15 102 (which is clearly an integration of De-A-1 961 769 to which it explicitly refers) teaches that different ratios of the sweetening components are necessary also in the case of sweetening very closely related compositions (i.e. beverages).

We have now found a particularly agreable and palatable solid, water-soluble, pharmaceutical composition containing the following parts by weight of the following components :

5-25 acetylcysteine, 70-85 xylitol, 0.5-1.2 sodium saccharin bihydrated, 0.4-1.2 beta carotene, 4-7.5 flavors.

Futhermore, the flavor presence permits also to adequately mask the acetylcysteine odor.

The preferred embodiment according to the present invention is constituted by granules and the preferred preparation consists of small bags of a non-toxic material, compatible with the granules, impervious to air and humidity, containing the suitable dosage unit. A typical example of a suitable material for said bags is represented by coupled aluminium-polyethylene foils, the polyethylene being in contact with the dosage units. Said dosage units are predetermined by the pharmacologist and the physician according to known criteria, and each contains preferably 100, 200, 400, 600 mg of acetylcysteine.

The solid, water-soluble, pharmaceutical compositions according to the present invention are prepared by methods known to the technician in the field, who has also knowledge of the materials and apparatus required.

Typically, the preparation of a granular material according to the present invention is carried out by first granulating xylitol with a water solution containing ca. 20% beta carotene and 20% sodium saccharin bihydrated in a fluid bed granulator. To the thus obtained granules there are then added in an external phase, acetylcysteine and orange flavor consisting of deterpenized essential oils supported on arabic gum and malto-dextrines, the essential oils representing approximately 18% by wt.

The thus prepared granules are distributed in small aluminium-polyethylene bags in amounts corresponding to the predetermined dosage units.

Non-limitative examples of compositions according to the present invention are :

|  | mg | mg | mg | mg |
|---|---|---|---|---|
| Acetylcysteine | 100 | 200 | 400 | 600 |
| Xylitol | 1271 | 1171 | 1471 | 2166 |
| Sodium saccharin bihydrated | 15 | 15 | 15 | 20 |
| Beta carotene | 14 | 14 | 14 | 14 |
| Orange flavor | 100 | 100 | 100 | 200 |

The pharmaceutical compositions according to the present invention have proved to be chemically as well as organoleptically stable for at least two years at room temperature.

The palatability tests have shown that the organoleptic characteristics of the compositions according to the present invention are significantly better than the ones possessed by the compositions sweetened with sorbitol, aspartame and similar products.

## Claims

1. A solid, water-soluble, pharmaceutical composition containing the following parts by weight of the following components :

5-25 acetylcysteine, 70-85 xylitol, 0.5-1.2 sodium saccharin bihydrated, 0.4-1.2 beta carotene and 4-7.5 flavors.

2. A pharmaceutical composition according to claim 1, in which each dosage unit consists of water-soluble granules having the following composition : 100 mg acetylcysteine, 1271 mg xylitol, 15 mg sodium saccharin bihydrated, 14 mg beta carotene, 100 mg orange flavor.

3. A pharmaceutical composition according to claim 1, in which each dosage unit consists of water-soluble granules having the following composition : 200 mg acetylcysteine, 1171 mg xylitol, 15 mg sodium saccharin bihydrated, 14 mg beta carotene and 100 mg orange flavor.

4. A pharmaceutical composition according to claim 1, in which each dosage unit consists of water-soluble granules having the following composition : 400 mg acetylcysteine, 1471 mg xylitol, 15 mg sodium saccharin bihydrated, 14 mg beta carotene and 100 mg orange flavor.

5. A pharmaceutical composition according to claim 1, in which each dosage unit consists of water-soluble granules having the following composition : 600 mg acetylcysteine, 2166 mg xylitol, 20 mg sodium saccharin bihydrated, 14 mg beta carotene, 200 mg orange flavor.

## Patentansprüche

1. Feste wasserlösliche pharmazeutische Zusammensetzung, enthaltend die folgenden Bestandteile in Gewichtsteilen : 5-25 Acetylcystein, 70-85 Xylitol, 0,5-1,2 Natriumsaccharin-Bihydrat, 0,4-1,2 beta-Karoten und 4-7,5 Geschmacksstoffe.

2. Pharmazeutische Zusammensetzung nach Anspruch 1, bei der jeweils die Einheitsdosis aus wasserlöslichen Kügelchen besteht mit der folgenden Zusammensetzung : 100 mg Acetylcystein, 1.271 mg Xylitol, 15 mg Natriumsaccharin-Bihydrat, 14 mg beta-Karoten, 100 mg Orangengeschmacksstoff.

3. Pharmazeutische Zusammensetzung nach Anspruch 1, bei der jeweils die Einheitsdosis aus wasserlöslichen Kügelchen besteht mit der folgenden Zusammensetzung : 200 mg Acetylcystein, 1.171 mg Xylitol, 15 mg Natriumsaccharin-Bihydrat, 14 mg beta-Karoten und 100 mg Orangengeschmacksstoff.

4. Pharmazeutische Zusammensetzung nach Anspruch 1, bei der jeweils die Einheitsdosis aus wasserlöslichen Kügelchen besteht mit der folgenden Zusammensetzung : 400 mg Acetylcystein, 1.471 mg Xylitol, 15 mg Natriumsaccharin-Bihydrat, 14 mg beta-Karoten und 100 mg Orangengeschmacksstoff.

5. Pharmazeutische Zusammensetzung nach Anspruch 1, bei der jeweils die Einheitsdosis aus wasserlöslichen Kügelchen besteht mit der folgenden Zusammensetzung : 600 mg Acetylcystein, 2.166 mg Xylitol, 20 mg Natriumsaccharin-Bihydrat, 14 mg beta-Karoten und 200 mg Orangengeschmacksstoff.

**Revendications**

1. Composition pharmaceutique solide, hydrosoluble, contenant les composants suivants par parties en poids : 5 à 25 parties d'acétylcystéine, 70 à 85 parties de xylitol, 0,5 à 1,2 parties de β-carotème et 4 à 7,5 parties d'arômes.

2. Composition pharmaceutique selon la revendication 1, dans laquelle chaque dose unitaire consiste en granules hydrosolubles ayant la composition suivante : 100 mg d'acétylcystéine, 1271 mg de xylitol, 15 mg de saccharine de sodium dihydratée, 14 mg de β-carotène, 100 mg d'arôme d'orange.

3. Composition pharmaceutique selon la revendication 1, dans laquelle chaque dose unitaire consiste en granules hydrosolubles ayant la composition suivante : 200 mg d'acétylcystéine, 1171 mg de xylitol, 15 mg de saccharine de sodium dihydratée, 14 mg de β-carotène et 100 mg d'arôme d'orange.

4. Composition pharmaceutique selon la revendication 1, dans laquelle chaque dose unitaire consiste en granules hydrosolubles ayant la composition suivante : 400 mg d'acétylcystéine, 1471 mg de xylitol, 15 mg de saccharine de sodium dihydratée, 14 mg de β-carotène et 100 mg d'arôme d'orange.

5. Composition pharmaceutique selon la revendication 1, dans laquelle chaque dose unitaire consiste en granules hydrosolubles ayant la composition suivante : 600 mg d'acétylcystéine, 2166 mg de xylitol, 20 mg de saccharine de sodium dihydratée, 14 mg de β-carotène et 200 mg d'arôme d'orange.